# EUROPÄISCHE PATENTANMELDUNG

(11) **EP 1 219 284 A1**
(43) Veröffentlichungstag der Anmeldung: **03.07.2002**
(21) Anmeldenummer: 00126872.1
(22) Anmeldetag: 07.12.2000
(51) Int. Cl.: A61K 6/00

(54) **Wässrige Zusammensetzung mit plaquehemmender Wirkung auf weichbleibenden, auf Silikon basierenden Zahnprothesenunterfütterungen**

(71) Anmelder: DETAX Karl Huber GmbH & Co.KG, 76275 Ettlingen (DE)
(72) Erfinder: Sohr, Volker, Dr., 76307 Karlsbad (DE); Juretzki, Ursula, 76185 Karlsruhe (DE)
(74) Vertreter: Winter, Brandl, Fürniss, Hübner, Röss, Kaiser, Polte Partnerschaft

(57) **Zusammenfassung**

Es wird eine wäßrige Zusammensetzung mit plaquehemmender Wirkung auf weichbleibenden, auf Silikon basierenden Zahnprothesen-Unterfütterungen vorgeschlagen, mit:
0,001 bis 5 Gew.-% Chlorhexidin oder eines oder mehrerer physiologisch akzeptabler Salze davon;
0,01 bis 30 Gew.-% eines oder mehrerer, die Benetzung von Silikonoberflächen fördernder Tenside;
0 bis 5 Gew.-% eines oder mehrerer weiterer antibakterieller Zusätze;
0 bis 2 Gew.-% eines oder mehrerer ätherischer Öle;
0 bis 10 Gew.-% eines oder mehrerer Emulgatoren;
0 bis 20 Gew.-% Ethanol;
0 bis 30 Gew.-% Glycerin;
0 bis 2 Gew.-% eines oder mehrerer Aromastoffe, insbesondere eines oder mehrerer Süßstoffe.

## Beschreibung

Die vorliegende Erfindung betrifft eine wäßrige Zusammensetzung mit plaquehemmender Wirkung auf weichbleibenden, auf Silikon basierenden Zahnprothesen-Unterfütterungen sowie die Verwendung dieser Zusammensetzung zur Hemmung der Plaquebildung.

Zahnprothesen werden zur Verbesserung des Tragekomforts, insbesondere der Paßgenauigkeit, der Haftung und der Mundverweildauer sowie zur Vermeidung von Druckstellen mit weichbleibenden Kunststoffen unterfüttert. In der Praxis haben sich hierfür auf Silikonen (insbesondere vom additionsvernetzten Typ) basierende Unterfütterungen durchaus bewährt, da sie permanent weichbleibend, schleimhautverträglich, geruchs- und geschmacksneutral, speichelresistent, biokompatibel und toxikologisch unbedenklich sind.

Bei manchen Patienten neigen allerdings solche, mit weichbleibenden Silikonen unterfütterte Zahnprothesen beim Tragen dazu, im Laufe der Zeit Plaque anzulagern (siehe M. Hinz, R. Göbel, D. Welker: "Weiche Kunststoffe für Prothetik und Epithetik", Quintessenz Zahntech. 26,5, S. 491-499(2000)), was dann einerseits ein ästhetisches, andererseits ein oralhygienisches Problem darstellt. Zudem wird die Paßgenauigkeit der unterfütterten Prothese eingeschränkt.

Diese Plaqueanlagerung ist durch alleiniges Reinigen der Prothese nicht zu verhindern; sie tritt auch bei Prothesen, die mit handelsüblichen Mitteln desinfiziert wurden, nach einiger Zeit erneut auf.

Ein wesentlicher Beitrag zur Bildung von Plaque ist die Anlagerung von Bakterien, insbesondere von *Streptococcus mutans*, aus der Mundflora auf die Silikonoberfläche.

Es ist eine ganze Reihe von antimikrobiellen Wirkstoffen für den Einsatz im Dentalbereich bekannt. Gängige antimikrobielle Wirkstoffe umfassen ätherische Öle, insbesondere in alkoholischer Lösung, Alkaloide, Antibiotika, Bis-Guanidine, Detergentien, Fluoride, Metallionen, Phenole und Polyalkohole. Nachteilig bei vielen dieser Wirkstoffe ist insbesondere ihre geringe Substantivität, d.h. ihre geringe Verweildauer am Wirkort.

Demgegenüber zeichnet sich Chlorhexidin, d.h. 1,1'-Hexamethylen-bis-[5-(4-chlorphenyl)-biguanid] gemäß der Formel I: 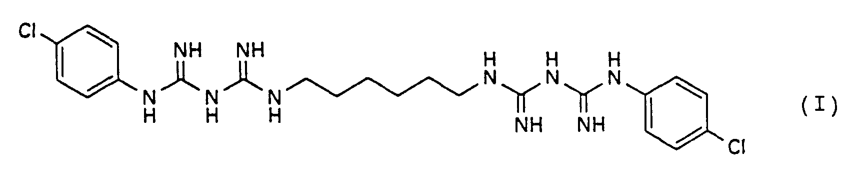 durch eine hohe Substantivität aus, da es sich an die Oberflächen der Mundhöhle anlagert und von diesen langsam wieder freigesetzt wird. Erhöhte Chlorhexidinwerte liegen noch 24 Stunden nach einmaligem Spülen vor. Chlorhexidin zeichnet sich zudem durch eine hohe Wirksamkeit insbesondere gegenüber *Streptococcus mutans* aus und kann somit einen wertvollen Beitrag zur Oralhygiene leisten.

Im Falle von mit weichbleibenden Silikonen unterfütterten Zahnprothesen steht bei einer oralen Anwendung von Chlorhexidinpräparaten, z.B. von üblichen Mundspüllösungen, am Wirkort, d.h. auf der Silikonoberfläche, in der Regel entweder keine ausreichende Wirkstoffkonzentration zur Verfügung oder es müßten derart hohe Chlorhexidinkonzentrationen zur Anwendung kommen, daß insbesondere bei einer Daueranwendung mit nachteiligen Nebenwirkungen zu rechnen wäre.

Auch die in jüngerer Zeit entwickelten, auf Chlorhexidin basierenden Lacke eignen sich in der Regel nicht zur Hemmung der Plaquebildung, da das weichbleibende Silikonmaterial nicht zur Lackierung geeignet ist und auch im Mundbereich am Wirkort keine lackierbaren Flächen zur Verfügung stehen.

Die Aufgabe der vorliegenden Erfindung bestand demnach darin, ein Mittel zur Hemmung der Plaquebildung auf weichbleibenden, auf Silikon basierenden Zahnprothesen-Unterfütterungen zur Verfügung zu stellen, das im wesentlichen die Nachteile des oben genannten Standes der Technik vermeidet. Insbesondere soll bei der Anwendung dieses Mittels das Bakterienwachstum auf der Silikonunterfütterung und somit die Bildung von Plaque gehemmt werden. Außerdem sollte sich das Mittel möglichst einfach, insbesondere als Spray anwenden lassen.

Die Lösung dieser Aufgabe wird mit den Merkmalen der unabhängigen Ansprüche erreicht. Die abhängigen Ansprüche geben bevorzugte Ausführungsformen der vorliegenden Erfindung an.

Gegenstand der vorliegenden Erfindung ist eine wäßrige Zusammensetzung mit plaquehemmender Wirkung auf weichbleibenden, auf Silikon basierenden Zahnprothesen-Unterfütterungen mit:
0,001 bis 5 Gew.-% Chlorhexidin oder eines oder mehrerer physiologisch akzeptabler Salze davon;
0,01 bis 30 Gew.-% eines oder mehrerer, die Benetzung von Silikonoberflächen fördernder Tenside;
0 bis 5 Gew.-% eines oder mehrerer weiterer antibakterieller Zusätze;
0 bis 2 Gew.-% eines oder mehrerer ätherischer Öle;
0 bis 10 Gew.-% eines oder mehrerer Emulgatoren;
0 bis 20 Gew.-% Ethanol;
0 bis 30 Gew.-% Glycerin;
0 bis 2 Gew.-% eines oder mehrerer Aromastoffe, insbesondere eines oder mehrerer Süßstoffe.

Chlorhexidin ist eine stark basisch reagierende Substanz, die in Wasser nur schwer löslich ist (0,06% bei 20°C). In der Praxis bevorzugt man Salze des Chlorhexidins mit physiologisch akzeptablen Säuren wie z.B. Chlorhexidindihydrochlorid, Chlorhexidindiacetat und insbesondere das gut wasserlösliche Chlorhexidindigluconat, welches als 20 %ige wäßrige Lösung in den Handel kommt. Vorzugsweise enthält die erfindungsgemäße Zusammensetzung 0,01 bis 2 Gew.-%, insbesondere 0,01 bis 0,2 Gew.-% Chlorhexidin(salz).

Eine gute und vollständige Benetzung der Silikonoberfläche der Zahnprothesen-Unterfütterung ist wesentlich für einen optimalen Kontakt der erfindungsgemäßen Zusammensetzung mit der bereits auf der Silikonoberfläche befindlichen Plaque. Geeignete Tenside können aus den im Dentalbereich üblichen anionischen, kationischen und nicht-ionischen Tensiden ausgewählt werden. Vorzugsweise sind in der erfindungsgemäßen Zusammensetzung 0,1 bis 10 Gew.-% eines oder mehrerer, die Benetzung von Silikonoberflächen fördernder Tenside enthalten.

Besonders bevorzugte Tenside sind polyalkylenoxidmodifizierte Polydimethylsiloxane, z.B. Polydimethylsiloxan-Polyethylenoxid-Copolymer (CAS-Nr. 68937-54-2). Diese zeichnen sich nicht nur durch eine hervorragende Verringerung der Oberflächenspannung der erfindungsgemäßen Zusammensetzung aus, so daß diese vorteilhaft als Spray formuliert werden kann, sondern auch durch eine hohe Affinität zu Silikonoberflächen, was eine optimale Benetzung derselben gewährleistet.

Die erfindungsgemäße Zusammensetzung enthält vorzugsweise weitere antibakterielle Zusätze, insbesondere Salze von Silber, Kupfer, Zinn oder Zink, Metallfluoride bzw. Aminhydrofluoride, Cetylpyridiniumchlorid, Benzalkoniumchlorid, Biozide wie Ciprofloxacin-HCl oder -Betain, usw. Da es bei vielen Metallionen zur Bildung eines dunklen Belags kommen kann, weil diese Metallionen mit den Thiolgruppen der Proteine reagieren können und schwerlösliche Sulfide bilden können, werden Zinksalze besonders bevorzugt, da Zinksulfid weiß oder hellgrau ausfällt.

Diese weiteren antibakteriellen Zusätze können die Wirkung des prinzipiellen Wirkstoffs Chlorhexidin in der erfindungsgemäßen Zusammensetzung synergistisch unterstützen. Vorzugsweise enthält die erfindungsgemäße Zusammensetzung 0,001 bis 5 Gew.-%, insbesondere 0,01 bis 0,2 Gew.-% dieser weiteren antibakteriellen Zusätze

Die erfindungsgemäße Zusammensetzung enthält vorzugsweise auch ätherische Öle, insbesondere solche mit antimikrobieller Wirkung, z.B. Zitrusöle, Minzöle wie z.B. Pfefferminzöl, Krauseminzöl, Wintergrünöl, usw., bzw. ätherische Substanzen wie Thymol, Eukalyptol, Methylsalicylat und Menthol. Vorzugsweise sind in der erfindungsgemäßen Zusammensetzung 0,001 bis 2 Gew.-%, insbesondere 0,01 bis 0,5 Gew.-% ätherische Öle enthalten.

Da die prinzipiellen Bestandteile der erfindungsgemäßen Zusammensetzung wasserlöslich sind, ist die Verwendung von Emulgatoren in den meisten Fällen nicht zwingend erforderlich. Werden indessen größere Mengen ätherischer Öle oder unlösliche bzw. die Löslichkeit herabsetzende Bestandteile eingesetzt, kann die Verwendung eines oder mehrerer Emulgatoren sinnvoll sein. Als Emulgatoren können die üblichen Öl-in-Wasser-Emulgatoren eingesetzt werden, insbesondere Polyoxyethylenfettsäureester wie z.B. polyethoxyliertes hydriertes Rizinusöl (Macrogolglycerolhydroxysterat) oder Polyoxyethylensorbitanfettsäureester, usw. Vorzugsweise sind in der erfindungsgemäßen Zusammensetzung 0,01 bis 10 Gew.-%, insbesondere 0,05 bis 2 Gew.-% Emulgatoren enthalten.

Die erfindungsgemäße Zusammensetzung enthält vorzugsweise auch Ethanol, da dieses eine die Bakterienzellwand denaturierende Wirkung aufweist und dadurch die Wirkung des Chlorhexidins synergistisch unterstützen kann. Vorzugsweise liegt der Ethanolgehalt im Bereich von 0,1 bis 20 Gew.-%, insbesondere zwischen 1 und 15 Gew.-%.

Weiterhin enthält die erfindungsgemäße Zusammensetzung vorzugsweise auch Glycerin, da dieses die Viskosität erhöht und daher die Anhaftung der Zusammensetzung auf der Silikonoberfläche verbessern kann. Ein weiterer Vorteil bei der Verwendung von Glycerin besteht darin, daß es aufgrund seiner Hygroskopie als Feuchthaltemittel wirkt und daher bei der Anwendung der erfindungsgemäßen Zusammensetzung als Pumpspray in der Pumpsprayflasche eine Verstopfung der Düse durch Austrocknung verhindert. Der Glyceringehalt liegt vorzugsweise im Bereich von 1 bis 30 Gew.-%, insbesondere zwischen 1 und 10 Gew.-%.

Aromastoffe sind zur Erzielung der plaquehemmenden Wirkung der erfindungsgemäßen Zusammensetzung nicht erforderlich. Sie können indessen die Akzeptanz der Zusammensetzung durch den Anwender verbessern. Als Aromastoffe können alle auf dem Dentalgebiet üblichen Aromastoffe verwendet werden, sofern sie mit der erfindungsgemäßen Zusammensetzung kompatibel sind, insbesondere Süßstoffe wie z.B. Aspartam, Saccharin-Na oder Natriumcyclamat, vorzugsweise in Mengen von 0,001 bis 1 Gew.-%, insbesondere 0,01 bis 0,1 Gew.-%.

Ein weiterer Gegenstand der vorliegenden Erfindung ist die Verwendung der vorstehend beschriebenen wäßrigen Zusammensetzung zur Hemmung der Plaquebildung auf weichbleibenden, auf Silikon basierenden Zahnprothesen-Unterfütterungen.

In der Praxis kann die Zusammensetzung auf die Silikonunterfütterung aufgetropft, bevorzugt aber aufgesprüht werden (mit Treibgas oder als Pumpspray). Dies sollte vorzugsweise zumindest einmal täglich geschehen. Die Prothese wird danach im feuchten Zustand wieder eingesetzt.

Ohne an eine bestimmte Theorie gebunden sein zu wollen, wird vermutet, daß zusätzlich zur unmittelbaren Einwirkung des Chlorhexidins auf die auf der Silikonoberfläche vorhandene Plaque (Desinfizierung) auch eine reversible Bindung und langsame Freisetzung des Wirkstoffs an bzw. aus der mit der Silikonunterfütterung der Prothese in Berührung stehenden Mundschleimhaut eintritt, so daß eine ausreichend hohe Chlorhexidinkonzentration über einen längeren Zeitraum direkt am Wirkort gewährleistet ist.

Die Erfindung wird nachstehend anhand von Beispielen und Abbildungen näher erläutert, welche rein illustrativ sind und keine Beschränkung des Umfangs der vorliegenden Erfindung darstellen sollen.

Es zeigt:
Fig. 1 eine mikroskopische Aufnahme eines Abstrichs ohne die Verwendung der erfindungsgemäßen Zusammensetzung.
Fig. 2 eine mikroskopische Aufnahme eines Abstrichs unter Verwendung der erfindungsgemäßen Zusammensetzung.

### Beispiel 1 (Formulierungsbeispiel)

| | | |
|---|---|---|
| (1) | 0,20 g | Chlorhexidindigluconat-Lösung (20 Gew.-% in Wasser) |
| (2) | 0,25 g | Macrogolglycerolhydroxystearat |
| (3) | 11,50 g | Ethanol (86 Gew.% ) |
| (4) | 0,055 g | Pfefferminzöl |
| (5) | 0,045 g | Krauseminzöl |
| (6) | 0,10 g | Menthol |
| (7) | 4,00 g | Glycerin (85 Gew.%) |
| (8) | 0,02 g | Saccharin-Na |
| (9) | 2,00 g | Polydimethylsiloxan-Polyethylen-oxid-Copolymer (MG ca. 3000) |
| (10) | ad 100 g | gereinigtes Wasser |

50 g Wasser werden vorgelegt und die Komponenten (2) und (9) darin aufgelöst. Unter Rühren wird anschließend eine Lösung der Komponenten (4)-(7) in (3) langsam zugegeben. Nach Einrühren der Bestandteile (1) und (8) wird unter Rühren mit gereinigtem Wasser auf 100 g Lösung verdünnt. Es wird eine homogene, opaleszierende Flüssigkeit erhalten.

### Beispiel 2 (Anwendungsbeispiel)

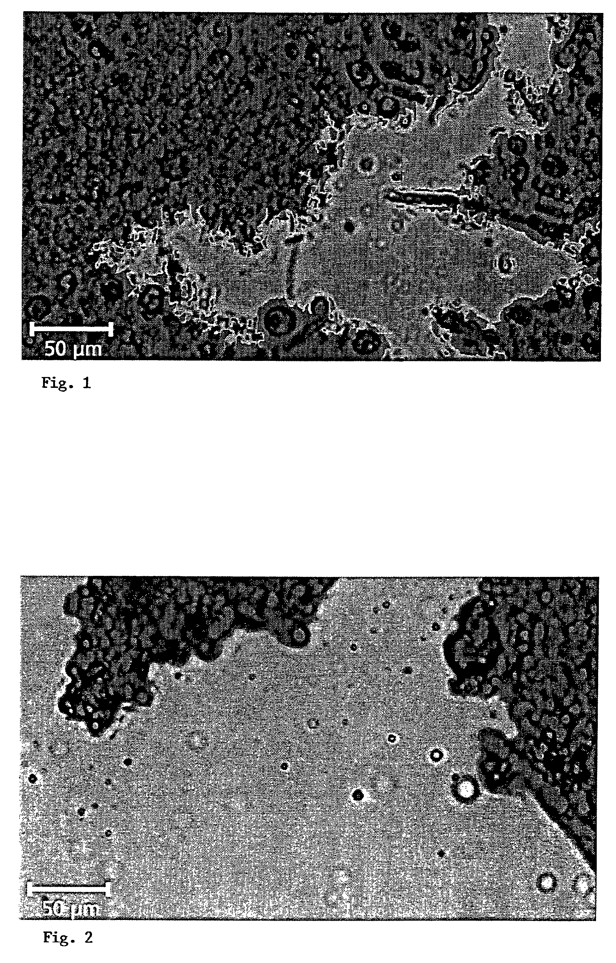

Das Ausmaß der Plaquehemmung wurde durch mikroskopische Untersuchung von Prothesenabstrichen nachgewiesen. Fig.1 zeigt eine mikroskopische Aufnahme eines Abstriches einer mit dem Unterfütterungssilikon Mollosil® plus (additionsvernetztendes Silikon der Fa. DETAX GmbH & Co. KG, Ettlingen, Deutschland) unterfütterten Oberkiefer-Prothese nach einer Tragedauer von 24 Std. Nach Aufsprühen des Pflegemittels auf die Siliconunterfütterung und Wiedereinsetzen der Prothese wurde nach 24 Std. Tragezeit ein erneuter Abstrich vorgenommen. Die mikroskopische Untersuchung dieses Abstriches (Fig. 2) zeigt eindeutig eine Verringerung der Keimzahl.

## Patentansprüche

1. Wäßrige Zusammensetzung mit plaquehemmender Wirkung auf weichbleibenden, auf Silikon basierenden Zahnprothesen-Unterfütterungen mit:
0,001 bis 5 Gew.-% Chlorhexidin oder eines oder mehrerer physiologisch akzeptabler Salze davon;
0,01 bis 30 Gew.-% eines oder mehrerer, die Benetzung von Silikonoberflächen fördernder Tenside;
0 bis 5 Gew.-% eines oder mehrerer weiterer antibakterieller Zusätze;
0 bis 2 Gew.-% eines oder mehrerer ätherischer Öle;
0 bis 10 Gew.-% eines oder mehrerer Emulgatoren;
0 bis 20 Gew.-% Ethanol;
0 bis 30 Gew.-% Glycerin;
0 bis 2 Gew.-% eines oder mehrerer Aromastoffe, insbesondere eines oder mehrerer Süßstoffe.

2. Zusammensetzung nach Anspruch 1, **dadurch gekennzeichnet, daß** sie 0,01 bis 2 Gew.-%, insbesondere 0,01 bis 0,2 Gew.-% Chlorhexidin oder eines oder mehrerer physiologisch akzeptabler Salze davon enthält.

3. Zusammensetzung nach Anspruch 1 oder 2, **dadurch gekennzeichnet, daß** sie 0,1 bis 10 Gew.-% eines oder mehrerer, die Benetzung von Silikonoberflächen fördernder Tenside enthält.

4. Zusammensetzung nach einem der Ansprüche 1 bis 3, **dadurch gekennzeichnet, daß** sie 0,001 bis 5 Gew.-%, insbesondere 0,01 bis 0,2 Gew.-% eines oder mehrerer weiterer antibakterieller Zusätze enthält

5. Zusammensetzung nach einem der Ansprüche 1 bis 4, **dadurch gekennzeichnet, daß** sie 0,001 bis 2 Gew.-%, insbesondere 0,01 bis 0,5 Gew.-% eines oder mehrerer ätherischer Öle enthält

6. Zusammensetzung nach einem der Ansprüche 1 bis 5, **dadurch gekennzeichnet, daß** sie 0,01 bis 10 Gew.-%, insbesondere 0,05 bis 2 Gew.-% eines oder mehrerer Emulgatoren enthält.

7. Zusammensetzung nach einem der Ansprüche 1 bis 6, **dadurch gekennzeichnet, daß** sie 0,1 bis 20 Gew.-%, insbesondere 1 bis 15 Gew.-% Ethanol enthält.

8. Zusammensetzung nach einem der Ansprüche 1 bis 7, **dadurch gekennzeichnet, daß** sie 1 bis 30 Gew.-%, insbesondere 1 bis 10 Gew.-% Glycerin enthält.

9. Zusammensetzung nach einem der Ansprüche 1 bis 8, **dadurch gekennzeichnet, daß** sie 0,001 bis 1 Gew.-%, insbesondere 0,01 bis 0,1 Gew.-% eines oder mehrerer Aromastoffe, insbesondere eines oder mehrerer Süßstoffe enthält.

10. Verwendung der Zusammensetzung nach irgend einem der vorstehenden Ansprüche zur Hemmung der Plaquebildung auf weichbleibenden, auf Silikon basierenden Zahnprothesen-Unterfütterungen.
